Europäisches Patentamt

European Patent Office

Office européen des brevets

(19)

(11) Veröffentlichungsnummer: **0 164 069**
**B1**

(12) **EUROPÄISCHE PATENTSCHRIFT**

(45) Veröffentlichungstag der Patentschrift:
29.03.89

(21) Anmeldenummer: 85106648.0

(22) Anmeldetag: 30.05.85

(51) Int. Cl.⁴: **C 12 P 21/02,** A 61 K 45/02,
C 07 K 3/12

(54) Verfahren zur Isolierung und Reinigung von alpha-Interferonen.

(30) Priorität: 08.06.84 DE 3421302

(43) Veröffentlichungstag der Anmeldung:
11.12.85 Patentblatt 85/50

(45) Bekanntmachung des Hinweises auf die Patenterteilung:
29.03.89 Patentblatt 89/13

(84) Benannte Vertragsstaaten:
AT BE CH DE FR GB IT LI LU NL SE

(56) Entgegenhaltungen:
WO-A-83/03103
FR-A-2 266 741

CHEMICAL ABSTRACTS, Band 91, Nr. 23, 3.
Dezember 1979, Seite 461, Zusammenfassung Nr.
191106a, Columbus, Ohio, US; J.S. ERICKSON et
al.: "Purification of acid ethanol-extracted human
lymphoid interferons by Blue Sepharose
chromatography", & ANAL. BIOCHEM. 1979, 98(1),
214-18
NATURE, Band 294, Nr. 5838, 19. November 1981,
Seiten 278-280, Macmillan Journals Ltd, Chesham,
Bucks, GB; H. ARNHEITER et al.:
"Physicochemical and antigenic properties of
synthetic fragments of human leukocyte
interferon"

(73) Patentinhaber: HOECHST AKTIENGESELLSCHAFT,
Postfach 80 03 20, D-6230 Frankfurt am Main 80
(DE)

(72) Erfinder: Obermeier, Rainer, Dr., Langenhainer
Weg 14, D-6234 Hattersheim am Main (DE)
Erfinder: Salomon, Ingeborg, Taunusring 26, D-6362
Wöllstadt (DE)
Erfinder: Ludwig, Jürgen, Ringstrasse 13, D-6486
Brachttal (DE)

## Beschreibung

Bereits 1956 entdeckten Isaacs und Lindemann Interferon als einen indirekten Hemmstoff der intracellulären Virusvermehrung. Inzwischen wurde eine große Anzahl verschiedener Interferontypen natürlicher Herkunft identifiziert, die vor allem durch die unterschiedlichen Herkunftszellen charakterisiert werden:

α-Interferone aus Leukozyten
β-Interferone aus Fibroblasten
γ-Interferone aus Lymphozyten.

Die durch entsprechende Stimuli in den jeweiligen Zellen induzierten Interferone gehören chemisch zur Gruppe der Glykoproteine, sind z. T. säurestabil (pH 2) und entfalten ihre volle antivirale Wirkung von $10^8$ - $10^9$ IE/mg speziesspezifisch. Die Primärstruktur einer Reihe von α-, β- und γ-Interferonen, die aus 146 - 166 Aminosäuren aufgebaut sind, ist aufgeklärt. Daneben sind nicht in der Natur vorkommende Interferone bekannt geworden, deren Struktur auf gentechnologischem Wege entweder verkürzt, oder gegenüber den natürlichen Interferonen in der Aminosäuresequenz abgeändert wurden.

Jüngste Arbeiten auf dem Gebiet der Gentechnologie, bei denen Interferon-Gene in E. coli zur Expression gebracht wurden, haben gezeigt, daß die in den natürlichen Interferonen nachgewiesenen Glycosyl- bzw. Polysaccharid-Seitenketten keinen Einfluß auf die biologische Aktivität der Proteine zu haben scheinen. Darüberhinaus kann mit Antikörpern, die mit dem natürlichen Glykoprotein erzeugt wurden, eine vergleichbare Bindung an nicht glykosylierte Interferone aus E. coli gemessen werden, was auf den überwiegenden Einfluß der Proteinstruktur des Interferons als Antigendeterminante hindeutet.

Wegen ihrer therapeutischen Potenz werden die Interferone im großtechnischen Maßstab entweder durch Ernten von Kulturmedien entsprechender Zellkulturen, oder neuerdings durch Fermentation von E. coli-Stämmen , in die auf gentechnologischem Wege ein Interferoncodierender DNA-Vektor cloniert wurde produziert. Zur Isolierung und Reinigung der gewonnenen Rohinterferon-Präparate (durchschnittliche Aktivität $10^3$ - $10^4$ IE/mg Protein) werden im allgemeinen Verfahren der Affinitäts- und Adsorptionschromatographie verwendet. Diese nützen die spezielle Fähigkeit des Interferons an immobilisierte hydrophobe Liganden, Metallionen, Thiole, organische Quecksilberverbindungen, Polynukleotide, Controlled Pore Glass, sowie an Antikörper mit hoher Spezifität zu binden. Trotz der Vielfalt von Reinigungsmethoden bleiben Ausbeuten und Reinheit der damit erhaltenen Interferone jedoch unbefriedigend, wie die Unsicherheit über die Ursache der Nebenwirkungen bei der klinischen Prüfung der Interferone am Menschen zeigt.

Neue, effektive Isolierungsverfahren sind deshalb notwendig. Diese Aufgabe wird durch die vorliegende Erfindung eines Verfahrens zur Anreicherung und Isolierung von Interferon gelöst, das dadurch gekennzeichnet ist, daß man interferonhaltige gefriergetrocknete Rohgemische in einer wäßrigen Lösung von Harnstoff, die ein Tensid wie Natriumdodecylsulfat (SDS) enthält, löst und aus dieser Lösung das Interferon durch extraktive Zweiphasen-Verteilung mit einem Gemisch aus n-Butanol/Eisessig/$H_2O$ gewinnt. Dabei reichert sich das Interferon in der butanolhaltigen Phase an und kann daraus nach Verdünnen mit Wasser und Dialyse durch anschließende Gefriertrocknung isoliert werden. Das erfindungsgemäße Verfahren ist den bekannten Affinitäts- und Adsorptionsverfahren dadurch überlegen, daß es in einem effektiven, einstufigen Anreicherungsschritt die Eliminierung von mehr als 95 % aller begleitenden Verunreinigungen aus der E. coli-Fermentation ermöglicht.

Die Erfindung betrifft somit ein Verfahren zur Isolierung und Reinigung von plasmidogenen α-Interferonen aus gentechnologisch veränderten Bakterienkulturen und α-Interferonen aus Kulturüberständen von induzierten Säugetierzellen, das dadurch gekennzeichnet ist, daß man entsprechende interferonhaltige Rohsubstanzen zwischen einer tensidhaltigen wäßrigen Harnstofflösung und einer wasserhaltigen n-Butanol/Eisessig-Mischung verteilt und die gereinigten α-Interferone aus der oberen Phase isoliert.

Mit Hilfe des erfindungsgemäßen Verfahrens werden vorzugsweise Gemische aus plasmidogenen α-Interferonen (darunter werden in diesem Zusammenhang auch α-Hybridinterferone verstanden) und den bei seiner Gewinnung aus Bakterienaufschlüssen und Fermenterbrühen anfallenden Verunreinigungen getrennt.

Als zweiphasiges organisch-wäßriges Lösungsmittelsystem eignet sich besonders eine wäßrige Unterphase, die 1 bis 8 Mol Harnstoff/l und ein Aniontensid, vorzugsweise ein Alkylsulfat, insbesondere Natriumdodecylsulfat (SDS) beispielsweise in einer Konzentration von 0,1 - 2 Gew.-% enthält und wäßrig-organische Oberphase aus n-Butanol/Eisessig, vorzugsweise 300 bis 400 Volumenteile n-Butanol und 30 bis 50 Volumenteile Eisessig, insbesondere etwa 350 Volumenteile n-Butanol und 40 Volumenteile Eisessig, die bevorzugt mit Wasser gesättigt ist.

Das so in die Oberphase extrahierte Interferon kann danach durch Verdünnen mit Wasser und Entsalzen durch Dialyse und Gefriertrocknung isoliert werden.

Soll die Präparation noch weiter gereinigt werden, kann die interferonhaltige Oberphase beispielsweise einer weiteren Verteilung, vorzugsweise gegen eine n-Butanol und Eisessig enthaltende organisch-wäßrige Phase unterworfen werden. Als besonders geeignet haben sich Lösungsmittelsysteme folgender Zusammensetzung erwiesen:

n-Butanol: 200 bis 230, vorzugsweise etwa 215
Volumenteile
Eisessig: 165 bis 185, vorzugsweise etwa 175
volumenteile
Wasser: 2000 bis 2300, vorzugsweise etwa
2150 Volumenteile.

Die weitere Reinigung kann beispielsweise mittels einer Droplet-Countercurrent-Verteilung gegen das oben genannte wäßrige System als stationäre Phase erfolgen.

Eine weitere Möglichkeit an Hochreinigung ist Chromatographie über eine Verteilungssäule, die z. B. mit Sephadex[R] LH 20, äquilibriert z. B. mit n-Butanol/Eisessig/$H_2O$ (ca. 215 : 175 : 2150, Vol.:Vol.), gefüllt ist. Dazu wird die interferonhaltige Oberphase aus der Extraktion unmittelbar auf die Verteilungssäule aufgegeben und die Säule mit frischer Oberphase entwickelt.

Besonders vorteilhaft ist das erfindungsgemäße Verfahren zur Abtrennung von α-Interferonen aus Fermentationen von gentechnologisch modifizierten E. coli-Stämmen anzuwenden.

## Ausführungsbeispiele:

### Beispiel 1

Das Lysat einer 10 l Fermentation eines E. coli-Stammes, der plasmidogenes α-Interferon produziert, wird zentrifugiert. Der klare Überstand wird anschließend gefriergetrocknet. Ausbeute 13 g.

Der mit Hilfe der Reduktion des cytoplathischen Effekts (Lin et al. J. Gen. Virol. 39, 125 - 130, 1978) gemessene Interferongehalt dieses Materials beträgt $2 \cdot 10^8$ IFE/g Substanz.

10 g des Rohmaterials werden in 50 ml wäßrigen 6 M Harnstofflösung (1 % SDS) unter kräftigem Rühren suspendiert und der unlösliche Anteil abzentrifugiert. Die Lösung wird durch Zugabe von Eisessig auf ca. pH 3 eingestellt. Der entstandene Niederschlag wird nach 15-minütigem Rühren bei 4°C zentrifugiert und erneut in 50 ml 6 M Harnstofflösung (1 % SDS) aufgenommen. Die klare Lösung wird mit dem gleichen Volumen der Oberphase aus (n-Butanol/Eisessig 350 : 40; mit Wasser gesättigt) überschichtet. Die beiden Phasen werden 30 Minuten bei 4°C gut durchmischt und anschließend durch erneute Zentrifugation getrennt. Die Oberphase wird abgetrennt mit Wasser verdünnt, dialysiert und gefriergetrocknet. Der Rückstand (8 mg) besteht aus α-Interferon, das zu etwa 75 % rein ist und einen CPE-Wert von $2 \cdot 10^8$ IFE/mg Substanz aufweist. Dieses Material kann nun in üblicher Weise, z. B. über eine Affinitätssäule, an deren Trägermaterial kovalent Antiinterferon-Antikörper fixiert sind, chromatographiert werden. Der Reinheitsgrad beträgt danach mehr als 95 %. Das so gewonnene Interferon zeigt in der SDS-Gelelektrophorese eine einheitliche Bande.

### Beispiel 2

10 g Rohmaterial werden wie in Beispiel 1 durch Verteilung zwischen Oberphase/6 M Harnstofflösung (1 % SDS) aufgearbeitet. Die interferonhaltige Oberphase wird in einem DCC-Chromatographen (Droplet Countercurrent; z. B. Büchi 670) eingespeist. Die stationäre Phase des DCC-Chromatographen besteht aus n-Butanol/Eisessig/Wasser (215 : 175 : 2150 Volumenteile). Die Verteilung wird mit frischer Oberphase entwickelt. Das interferonhaltige Eluat wird mit einem Fraktionenkollektor gesammelt. Die entsprechenden Fraktionen werden vereinigt und das Interferon wie in Beispiel 1 durch Gefriertrocknung isoliert. Ausbeute 12 mg Interferon mit einem Gehalt von 63 %.

## Patentansprüche

1. Verfahren zur Isolierung und Reinigung von plasmidogenen α-Interferonen aus gentechnologisch veränderten Bakterienkulturen und α-Interferonen aus Kulturüberständen von induzierten Säugetierzellen, dadurch gekennzeichnet, daß man entsprechende interferonhaltige Rohsubstanzen zwischen einer tensidhaltigen wäßrigen Harnstofflösung und einer wasserhaltigen n-Butanol/Eisessig-Mischung verteilt und die gereinigten α-Interferone aus der oberen Phase isoliert.

2. Verfahren gemäß Anspruch 1, dadurch gekennzeichnet, daß α-Interferone aus gentechnologisch veränderten Bakterienkulturen isoliert und reinigt.

3. Verfahren gemäß Anspruch 2, dadurch gekennzeichnet, daß man α-Hybridinterferone isoliert und reinigt.

4. Verfahren gemäß einem der Ansprüche 1 bis 3, dadurch gekennzeichnet, daß die untere Phase 1 bis 8 Mol Harnstoff/l enthält.

5. Verfahren gemäß einem der Ansprüche 1 bis 4, dadurch gekennzeichnet, daß die untere Phase ein Aniontensid enthält.

6. Verfahren gemäß einem der Ansprüche 1 bis 5, dadurch gekennzeichnet, daß die untere Phase 0,1 bis 2 % Natriumdodecylsulfat enthält.

7. Verfahren gemäß einem der Ansprüche 1 bis 6, dadurch gekennzeichnet, daß die obere Phase ein wasserhaltiges Gemisch aus n-Butanol/Eisessig im Volumenverhältnis von etwa 350 : 40 ist.

8. Verfahren gemäß einem der Ansprüche 1 bis 7, dadurch gekennzeichnet, daß das n-Butanol/Eisessig-Gemisch mit Wasser gesättigt ist.

9. Verfahren gemäß einem der Ansprüche 1 bis 8, dadurch gekennzeichnet, daß die interferonhaltige Oberphase einer weiteren Verteilung gegen einer n-Butanol und Eisessig enthaltenden organisch-wäßrigen Phase unterwirft.

10. Verfahren gemäß Anspruch 9, dadurch gekennzeichnet, daß diese wäßrige Phase ein Gemisch aus n-Butanol/Eisessig/Wasser im Volumenverhältnis von etwa 215 : 175 : 2150 ist.

**Claims**

1. A process for the isolation and purification of α-interferons generated by plasmids from bacterial cultures which have been modified by genetic engineering, and from culture supernatants from induced mammalian cells, which comprises partition of appropriate crude substances containing interferon between an aqueous urea solution, which contains a surfactant, and a mixture of n-butanol and glacial acetic acid which contains water, and isolation of the purified α-interferons from the upper phase.

2. The process as claimed in claim 1, wherein α-interferons are isolated and purified from bacterial cultures which have been modified by genetic engineering.

3. The process as claimed in claim 2, wherein hybrid α-interferons are isolated and purified.

4. The process as claimed in one of claims 1 to 3, wherein the lover phase contains 1 to 8 mol/l urea.

5. The process as claimed in one of claims 1 to 4, wherein the lover phase contains an anionic surfactant.

6. The process as claimed in one of claims 1 to 5, wherein the lover phase contains 0.1 to 2 % sodium dodecyl sulfate.

7. The process as claimed in one of claims 1 to 6, wherein the upper phase is a mixture composed of n-butanol and glacial acetic acid, in the ratio by volume of about 350 : 40, which contains water.

8. The process as claimed in one of claims 1 to 7, wherein the mixture of n-butanol and glacial acetic acid is saturated with water.

9. The process as claimed in one of claims 1 to 8, wherein the upper phase containing interferon is subjected to a further partition between it and an organic/aqueous phase containing n-butanol and glacial acetic acid.

10. The process as claimed in claim 9, wherein this aqueous phase is a mixture composed of n-butanol/glacial acetic acid/water in the ratios by volume of about 215 : 175 : 2150.

**Revendications**

1. Procédé d'isolement et de purification d' α-interférons plasmidogènes à partir de cultures de bactéries modifiées par génie génétique et à partir des parties supérieures de cultures de cellules de mammifères induites, caractérisé en ce que l'on partage les substances brutes correspondantes contenant les interférons entre une solution aqueuse d'urée contenant un tensioactif et un mélange aqueux de n-butanol et d'acide adétique galical et on isole les α-interférons purifiés à partir de la phase supérieure.

2. Procédé selon la revendication 1, caractérisé en ce que l'on isole et purifie les α-interférons à partir de cultures de bactéries modifiées par génie génétique.

3. Procédé selon la revendication 2, caractérisé en ce que l'on isole et purifie des α-interférons hybrides.

4. Procédé selon l'une des revendications 1 à 3, caractérisé en ce que la phase inférieure contient de 1 à 8 moles d'urée par litre.

5. Procédé selon l'une des revendications 1 à 4, caractérisé en ce que la phase inférieure contient un tensioactif anionique.

6. Procédé selon l'une des revendications 1 à 5, caractérisé en ce que la phase inférieure contient de 0,1 à 2 % de dodécylsulfate de sodium.

7. Procédé selon l'une des revendications 1 à 6, caractérisé en ce que la phase supérieure est un mélange aqueux n-butanol/acide acétique glacial dans un rapport en volume d'environ 350 : 40.

8. Procédé selon l'une des revendications 1 à 7, caractérisé en ce que le mélange n-butanol/acide acétique glacial est saturé d'eau.

9. Procédé selon l'une des revendications 1 à 8, caractérisé en ce que l'on soumet la phase supérieure contenant l'interféron à un nouveau partage en utilisant une phase organo-aqueuse contenant du n-butanol et de l'acide acétique glacial.

10. Procédé selon la revendication 9, caractérisé en ce que cette phase aqueuse est un mélange n-butanol/acide acétique glacial/eau dans un rapport en volume d'environ 215 : 175 : 2150.